# EUROPEAN PATENT APPLICATION

(11) **EP 4 625 315 A1**
(43) Date of publication of application: **01.10.2025**
(21) Application number: 24167676.6
(22) Date of filing: 28.03.2024
(51) Int. Cl.: G06T 7/00

(54) **SYSTEMS AND METHODS FOR POLYP CLASSIFICATION**

(71) Applicant: Odin Medical Ltd., London EC2A 3AY (GB)
(72) Inventor: De Carvalho, Thomas, 94370 Sucy-en-Brie (FR); MOUNTNEY, Peter, London, SE23 2QU (GB); Brandao, Patrick, London, N4 3HT (GB)
(74) Representative: Gillott-Jones, Nathan Philip

(57) **Abstract**

A method of classifying a polyp captured in a tissue image of an in vivo tissue area is disclosed. The method includes a polyp during a colonoscopy procedure, analyzing, by a trained machine learned model, the tissue image, wherein the trained machine learned model is trained to identify classification characteristics of a polyp based on two or more visual characteristics, and generating a classification prediction of the tissue image based on the two or more visual characteristics including a basis of the classification prediction.

## Description

### FIELD

The described embodiments relate generally to a system for medical imaging systems, such as those for identifying polyps.

### BACKGROUND

Polyps are groupings of cells that may form on a lining of the colon or large intestine. Many polyps are harmless, but some polyps may develop into diseased or cancerous tissue. As a result, regular screening tests are often performed, such as a colonoscopy, to identify, diagnose, or remove polyps.

Commonly, diagnosis or identification of a polyp is performed by a medical professional after manually reviewing video or images capturing by a colonoscopy probe during or after a colonoscopy. However, accurately diagnosing benign or harmless polyps from diseased or cancerous polyps may be difficult by manual visual identification alone. As a result, patients are often subject to invasive diagnosis or removal methods, such as biopsies or polyp removal procedures as a precautionary measure, or diseased polyps may go undetected until the disease further progresses. Further, as a patient ages they commonly develop more polyps, resulting in even additional procedures.

Accordingly, there is a need for methods or systems to assist medical professionals to help identify and detect characteristics of a polyp accurately and quickly.

### SUMMARY

Embodiments of the present invention are directed to a classification of a polyp. In one example, a method of classifying a polyp is disclosed. The method includes capturing a tissue image of an in vivo tissue area including a polyp during a colonoscopy procedure, analyzing, by a trained machine learned model, the tissue image, wherein the trained machine learned model is trained to identify classification characteristics of a polyp based on two or more visual characteristics, and generating a classification prediction of the tissue image based on the two or more visual characteristics including a basis of the classification prediction.

In another example, a method of classifying a polyp is disclosed. The method includes receiving a tissue image of a tissue area including a polyp, analyzing, by a trained machine learned model, the tissue image, wherein the trained machine learned model is trained to identify classification characteristics of a polyp based on two or more visual characteristics, and generating a classification prediction of the tissue image based on the two or more visual characteristics including a basis of the classification prediction.

In some examples, the tissue image is captured during a colonoscopy procedure.

In some examples, the method includes determining the presence of the polyp in the tissue image.

In some examples, the classification characteristics include one or more polyp characteristics.

In some examples, the classification prediction includes a type classification for each polyp characteristic.

In some examples, the classification prediction includes a prediction of a type of polyp based on the two or more visual characteristics.

In some examples, the method includes validating the classification prediction to further train the trained machine learned model.

In some examples, the basis includes one or more of a confidence rating, a weighting of the classification characteristics, or a human readable description of the classification prediction.

In some examples, the trained machine learning model is a neural network.

In some examples, the classification characteristics of the polyp includes one or more polyp characteristics including one or more of a color of the polyp or a tissue area in the tissue image, vessel features of the polyp, or a surface pattern of the polyp.

In some examples, the classification prediction includes a recommended treatment including one or more of a biopsy of the polyp or a removal of the polyp.

In some examples, the method further includes training the machine learned model to classify features of the polyp.

In some examples, training the machine learned model includes receiving a plurality of training tissue images including a plurality of polyps.

In some examples, training the machine learned model includes identifying one or more of a plurality of visual characteristics within the training tissue images and labeling the training tissue images with the one or more of the plurality of visual characteristics to define labeled images.

In some examples, training the machine learned model includes providing the labeled images to a classifier to train the machine learned model to generate the trained machine learned model that can identify the classification characteristics of the polyp by the two or more visual characteristics.

In some examples, training the machine learned model includes training the machine learned model to recognize a presence of the polyp at the tissue area included in the training image.

In some examples of training the machine learned model, the two or more visual characteristics are multi-label classifications of the tissue images.

In some examples, training the trained machine learned model includes training the machine learned model to weight or prioritize one or more of the classification prediction of the polyp, at least one of the classification characteristics, or the two or more visual characteristics.

In some examples, each of the labeled images includes at least two or more visual characteristic labels identifying one or more classification characteristics by the plurality of visual characteristics.

In some examples, a set of the labeled images includes labels identifying the one or more polyp characteristics or the one or more type classifications associated with the one or more of the plurality of visual characteristics.

In some examples, the method is executed by a system including a probe comprising an imaging device, wherein the imaging device captures the tissue image of a tissue area including the polyp.

In some examples, the system includes a processing element forming a portion of the machine learned model to generate classification predictions, the machine learned model in operative communication with the probe.

In some examples, the method is executed by a system including a processing element forming a portion of the machine learned model to generate classification predictions, the machine learned model in operative communication with a probe comprising an imaging device configured to capture the tissue image. In some examples, the probe is separate from the system. In some examples, the captured tissue image is communicated to the system. In some examples, the captured tissue image is communicated to the system over a network.

In some examples of the methods, the classification prediction includes a weighting of the type prediction for each of the plurality of visual characteristics to generate a polyp type prediction.

In some examples, the classification prediction corresponds to a histopathological status of the polyp.

In some examples, the tissue image is captured at an in vivo location. For example, the tissue area is in vivo.

In some examples, the classification prediction includes two or more type classifications, each of the two or more type classifications based on separate sets of the two or more visual characteristics.

In one example, a computer-readable medium is disclosed. The computer-readable medium comprises instructions that, when executed by one or more processors of a computing device, cause the computing device to: receive a tissue image of a tissue area including a polyp, analyze, by a trained machine learned model, the tissue image, wherein the trained machine learned model is trained to identify classification characteristics of a polyp based on two or more visual characteristics; and generate a classification prediction of the tissue image based on the two or more visual characteristics including a basis of the classification prediction

In one example, a method for training a machine learned model to classify features of a polyp is disclosed. The method includes receiving a plurality of tissue images including a plurality of polyps, identifying two or more visual characteristics within the tissue images and labeling the tissue images with the two or more visual characteristics to define labeled images, providing the labeled images to a classifier to train the machine learned model, and generating a trained machine learned model that can identify classification characteristics of the polyp by the two or more visual characteristics.

In some examples, training the machine learned model includes training the machine learned model to recognize a presence of the polyp at an in vivo tissue area included in the training image.

In some examples, the machine learned model is a neural network model.

In some examples, the two or more visual characteristics are multi-label classifications of the tissue images.

In some examples, the method includes training the machine learned model to training the machine learned model to weight or prioritize one or more of a classification prediction of the polyp, at least one of the classification characteristics, or the two or more visual characteristics.

In one example, a system for classifying a polyp is disclosed. The system includes a probe including an imaging device, wherein the imaging device captures an image of a tissue area including the polyp and a processing element forming a portion of a trained machine learned model to generate classification predictions, wherein the trained machine learned model is in operative communication with the probe to receive the image and trained on labeled images of a plurality of polyps identified by one or more visual characteristics. The classification predictions are generated by identifying each of a plurality of visual characteristics of the polyp by the image, producing a classification prediction corresponding to the plurality of visual characteristics of the polyp, wherein the classification prediction includes a basis, and displaying the classification prediction.

In some examples of the system, the classification characteristics include one or more polyp characteristics.

In some examples of the system, the classification prediction includes a type classification for each polyp characteristic.

In some examples of the system, the classification prediction includes a prediction of a type of polyp based on the two or more visual characteristics.

In some examples of the system, the basis includes one or more of a confidence rating, a weighting of the classification characteristics, or a human readable description of the classification prediction.

In some examples of the system, the trained machine learning model is a neural network.

In some examples of the system, the classification prediction includes a recommended treatment including one or more of a biopsy of the polyp or a removal of the polyp.

In some examples of the system, a set of the labeled images includes labels identifying the one or more polyp characteristics or the one or more type classifications associated with the one or more of the plurality of visual characteristics.

In some examples of the system, the classification prediction includes two or more type classifications, each of the two or more type classifications based on separate sets of the two or more visual characteristics.

In some examples of the system, the one or more visual characteristics include one or more of a color, blood vessel features, or a surface pattern of the polyp.

In some examples of the system, the classification prediction includes a type prediction based on each of the plurality of visual characteristics.

In some examples of the system, the classification prediction includes a weighting of the type prediction for each of the plurality of visual characteristics to generate a polyp type prediction.

In some examples of the system, the polyp type prediction corresponds to a histopathological status of the polyp.

In some examples of the system, the tissue area is in vivo.

In some examples of the system, each of the labeled images includes at least two or more visual characteristic labels identifying one or more classification characteristics by the plurality of visual characteristics.

A number of feature refinements and additional features are applicable in the first aspect and contemplated in light of the present disclosure. These feature refinements and additional features may be used individually or in any combination. As such, each of the following features that will be discussed may be, but are not required to be, used with any other feature combination of the first aspect.

In addition to the exemplary aspects and embodiments described above, further aspects and embodiments will become apparent by reference to the drawings and by study of the following description.

### BRIEF DESCRIPTION OF THE DRAWINGS

The disclosure will be readily understood by the following detailed description in conjunction with the accompanying drawings, wherein like reference numerals designate like structural elements, and in which:
FIG. 1 is a schematic diagram of an example polyp classification system for detecting and classifying characteristics of a polyp;
FIG. 2 is a table showing information on various polyp characteristics and representative polyps organized by a type classification;
FIG. 3 is a schematic diagram of an example computer system as used by one or more devices of the example polyp classification system;
FIG. 4 depicts an example flowchart describing a method for training a system for classifying polyps; and
FIG. 5 depicts an example flowchart describing a method for detecting and classifying polyps.

### DETAILED DESCRIPTION

The description that follows includes sample systems, methods, and apparatuses that embody various elements of the present disclosure. However, it should be understood that the described disclosure may be practiced in a variety of forms in addition to those described herein.

The following disclosure relates generally to systems and methods for classifying polyps and polyp characteristics. The images of the polyps may be captured during a colonoscopy procedures, e.g., via a camera included as part of an endoscope. The system may capture and analyze in vivo tissue images from a colonoscopy to classify characteristics of a polyp. The system may generate classification predictions based on visual characteristics (e.g., two or more visual characteristics) of the polyp detected in the image. In some examples, the classification and characteristics analyzed by the system may based on the narrow band imaging (NBI) international colorectal endoscopic (NICE) classification system to predict a type classification of the polyp. By basing the analysis of the system on the NICE classification system, outputs to medical professionals may fit within their current diagnostic process.

The system may include one or more devices to analyze images of tissue to detect and classify, or to train for the detection of and classification of, polyps. The various devices may be in operative communication, directly or indirectly, through a network. The network may facilitate communication with or to the various devices.

The system may include a probe to capture images of tissue areas including a polyp. The probe may be an endoscopic probe or similar device and include an imaging device, such as a camera, to enable in vivo images of the intestinal tract or other internal tissue to be captured. In some examples, the probe may include two or more imaging devices and the devices may be configured to capture multiple frames at different positions or orientations within the body. In some embodiments, the probe may also include a light source, such as a light emitting diode (LED) to illuminate the tissue to enhance the images as captured, e.g., to allow accurate images that include color and other features that may not otherwise be visible in the images

The system may include a training database. The training database may include a plurality of labeled or training images of previously captured images of tissue areas and may include images including polyps. The labeled images may include labels for identifying characteristics of a polyp, such as by visual characteristics of the polyps. The images may be labeled individually for each of the characteristics or may include multiple labels for a plurality of characteristics. The labels may explain visual characteristics of the image. The labels may be metadata or natural language descriptions of the visual characteristics. In some examples, the labels may be automatically tagged to the images, or the images may be tagged by a medical professional. For example, an algorithm for that detects colors of pixels may be used to define a subset of labels of polyps in the labeled images.

The system may include a machine learned model. The machine learned model, which may be hosted or executed by the server, may be trained to recognize visual characteristics of the polyp to identify the characteristics of in vivo polyps. In some examples, the machine learned model may be a neural network (NN) model. The machine learned model may generate natural language outputs. The machine learned model may be trained on the labeled images of the training database.

The machine learned model generates classification predictions based on characteristics of polyps in the captured tissue images by the probe. The classification predictions may include a basis of the classification such as how each polyp characteristic indicates one or more type classification. Accordingly, the classification predictions may support or provide information to indicate a type classification or diagnosis of the polyp to assist a physician in making a diagnosis. By providing supporting information, the system may provide medical professionals with more flexible and comprehensive insight into a condition of a polyp. The classification predictions may be generated in real time by the machine learned model, such as during a colonoscopy procedure, or after a procedure.

Reference will now be made to the accompanying drawings, which assist in illustrating various features of the present disclosure. The following description is presented for purposes of illustration and description. Furthermore, the description is not intended to limit the inventive aspects to the forms disclosed herein. Consequently, variations and modifications commensurate with the following teachings, and skill and knowledge of the relevant art, are within the scope of the present inventive aspects.

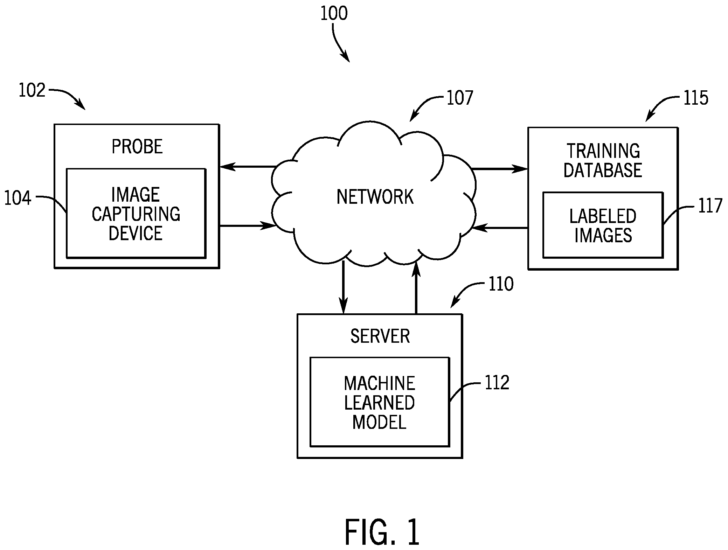

FIG. 1 depicts a schematic diagram of an example polyp classification system 100. The system may include one or more devices. For example, the system 100 may include a probe 102 or image device 104 to capture images of tissue, such as a camera. The system 100 may include a server 110 including a machine learned model 112. The polyp classification system 100 may facilitate training of, or analysis by, the machine learned model 112 to produce classification predictions of in vivo polyps to assist medical professionals in diagnosing a type of the polyp. The system 100 may include a training database 115 including information, such as training images 117, to train the machine learned model 112.

The system 100 may include or facilitate communication between the devices by a network 107. The network 107 may be wired or wireless and may be in direct or indirect communication with each of the devices. The network 107 may be formed by one or more computing devices, such as a second server.

The probe 102 may be a device to capture in vivo images of a tissue area. The tissue area may be in a body (e.g. in vivo) or a sample of the tissue. The probe 102 may be invasive or noninvasive. In one example, the probe 102 is an endoscopic probe to view or capture colon polyps. For example, the probe 102 may include an elongated portion or feature which may be inserted into a colon or intestine and view a length of the intestine.

The probe 102 may include an imaging or image capturing device 104. In one example, the imaging device 104 may be or defined by a videoscope or positioned at an end of the elongated feature of the probe 102. The image capturing device 104 may capture tissue images, such as photographs or video, of the tissue area. The image capturing device 104 may capture a variety of electromagnetic radiation such as visible light, infrared light, or other regions of the electromagnetic spectrum useful in identifying features of and classifying a polyp. In some examples, two or more imaging devices 104 may be used to capture multiple frames at different positions or viewing orientations of a tissue area in a body. The probe 102 or image capturing device 104 may include a light source, such as a light emitting diode (LED). The light source may illuminate a tissue area to enhance the quality of the image captured. For example, the light source may illuminate or make visible features of the tissue area. The light source may generate a variety of wavelengths of electromagnetic radiation. The region of the electromagnetic spectrum captured by the image capturing device 104 or the type of image capturing device 104 may correspond to the emissions from the light source. For example, the image capturing device 104 or light source may be selected to capture or filter wavelengths used by the classification system on which a diagnosis may be based. For example, the NICE classification system requires narrow band imaging. In such an example, the light is filtered into specific wavelengths that are absorbed differently by different tissue or tissue proteins, such as hemoglobin, to provide contrast between the tissue types. The filtering may be done by the light emitted from the light source, the types of light captured by the device, or by analyzing only data corresponding to the desired wavelengths of light. In some examples, images may be captured by a device separate from the system 100, such as a second probe or image capturing device. The captured tissue images may be communicated to the system 100, such as by the network 107, for use and analysis by the system 100.

The probe 102 may be guided by a medical professional to a tissue area including a polyp to capture the tissue images by the image capturing device 104. In some examples, the polyps may be automatically detected and tissue images may be captured by the image capturing device 104. In some examples, the image capturing device 104 may capture regions of the tissue (e.g. continuous or predetermined captures) and tissue images of polyps may be detected by the system 100 or the tissue images containing polyps may be identified at a later time. The probe 102 may be in communication with the various other devices, such as through the network 107, to transmit the captured tissue images of polyps.

In some examples, the probe 102 may include a tracking or locating tool to correlate a tissue image with an image location. For example, an external imaging device (e.g. X-Ray, CT, MRI, Ultrasound, or the like) may track a location of the image capturing device 104 to determine where tissue images are captured relative to a body of a patient. In some examples, optical information gathered by the probe 102 or image capturing device 104 may be used to map the tissue area and determine the image locations. In some examples, a length of probe 102, or similar device, inserted into the body may be used to determine an image location.

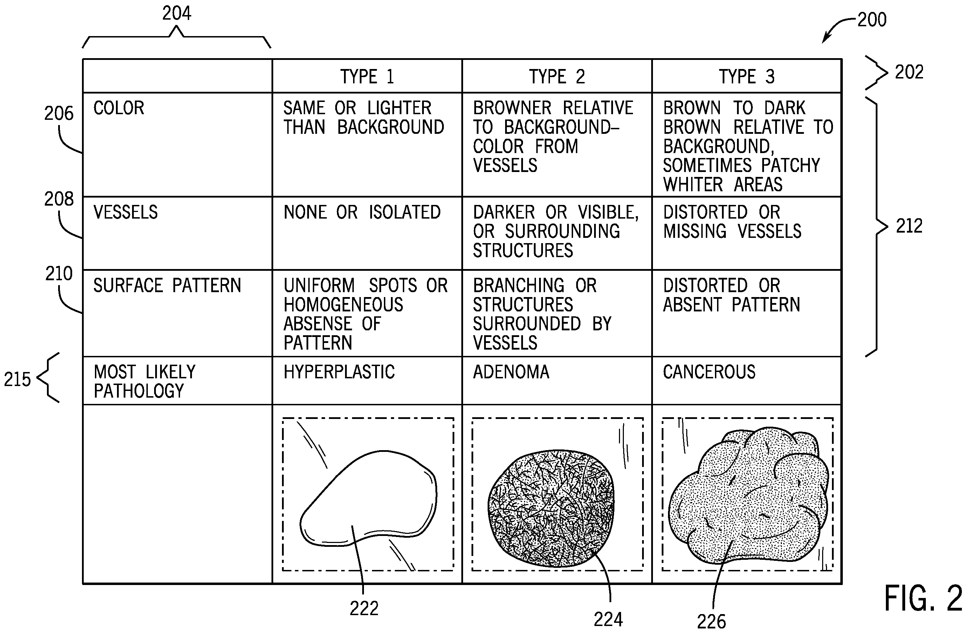

The training database 115 may be a computing device, such as a server, including or storing a plurality of training or labeled images 117 (e.g. labeled photos or videos). The labeled images 117 may be images of a plurality of polyps labeled by polyp characteristics 204, including visual characteristics 212 of the polyps, as shown in FIG. 2. The labeled images 117 may be images captured during previous procedures, such as by a probe 102 or image capturing device 104. In some examples, the labeled images 117 may be captured by a similar image device as used by the system 100, or may be filtered to provide similar visual data as that of the image capturing device 104. In some examples, the images may be sourced from other medical professionals or accessible databases, such as anonymized images. The captured images may then be labeled with information to define a labeled image 117 and stored, such as in the training database 115. In some examples, performing additional procedures, additional images may be captured and additional training or labeled images 117 may be generated and stored in the training database 115 for use.

Each labeled image 117 may be labeled to identify visual characteristics 212 of the polyps. The visual characteristics 212 may be associated with or correspond to polyp characteristics 204 indicative of a type classification 202 or histopathology 215 of the polyp. In some examples, each labeled image 117 may include two or more labels identifying two or more separate visual characteristics 212 and/or polyp characteristics 204 of a polyp. The labels of the polyps may also include type classifications 202 indicated by the visual characteristics 212 and/or polyp characteristics 204. In some examples, the labels may be or include metadata tags, natural language descriptions or human readable language of the visual characteristics 212 or polyp characteristics 204 as example explanations of the labeled features. The labels may be stored with the labeled image 117 (e.g. in the same file or storage location), or the labels may be stored separately from the images and linked to or otherwise associated with the images to define a labeled image 117. The labeled images 117 may also include corresponding treatment recommendations or actual treatments for the various training polyps in the images 117. Accordingly, the labeled images 117 may include varying types of labels conveying varying degrees of information about the visual characteristics 212. The labels may be generated by a medical professional reviewing the images for the visual characteristics 212 and assigning corresponding labels to the visual characteristics 212. In some examples, all or a portion of the labels may be generated automatically. For example, an algorithm that compares or analyzes pixels in images to detect color may assign labels to color related visual characteristics 212.

With continued reference to FIG. 1, the machine learned model 112, which may be hosted or executed by the server 110, is trained to analyze polyps captured in in vivo tissue images to generate classification predictions of the polyp. In one example, the machine learned model 112 is a neural network model. The machine learned model 112 may be a multi-label classifier. For example, the machine learned model 112 may generate outputs or predictions based on two or more analyzed characteristics of an image. In some examples, the machine learned model 112 may generate two or more outputs based on one or more analyzed characteristics. For example, the machine learned model 112 may be multi-headed and generate two or more independent or related outputs. In one example, the multiple outputs may include an overall prediction based on the analyzed characteristic and a basis or description of how that characteristic influences the overall prediction. The multiple outputs or predictions may be compared against each other to further improve or refine the predictions.

The machine learned model 112 is trained on the labeled images 117 to detect or determine the polyp characteristics 204 or type classifications 202 by the visual characteristics 212 of a polyp. For example, the machine learned model 112 may be trained to detect or associate the visual characteristics 212 of a polyp with the polyp characteristics 204, or visual characteristics 212 and polyp characteristics 204 with type classifications 202. In some examples, the association may be defined by labeled images 117 including labels for the visual characteristics 212 and one or more of the polyp characteristics 204 or type classifications 202. In some examples, the association may be generated after a detected pattern is determined in the labeled images 117 or between a plurality of labeled images 117 by the machine learned model 112 during training.

The machine learned model 112 may be trained to weight various visual characteristics 212 over other visual characteristics 212. For example, the labeled images 117 may include information indicating whether certain characteristics 212, or combinations of visual characteristics 212, are more indicative of a polyp characteristic 204 or type classification 202. In such an example, the machine learned model 112 may develop a weighting factor or priority order for the various visual characteristics 212. In some examples, the machine learned model 112 may generate multiple outputs, or classification predictions, and weight the classifications. For example, the machine learned model may weight each classification prediction by the likelihood each or two or more of the multiple predictions are indicative of the actual classification compared to each other or another of the predictions.

The machine learned model 112 may be trained to assign a confidence rating to the detected or determined features of the polyp in a tissue image. For example, the machine learned model 112 may assign a confidence rating to the detected visual characteristics 212. The confidence rating may correspond to a similarity between the detected visual characteristic 212 and corresponding (e.g. similar or matching) visual characteristics 212 in the labeled images 117. In some examples, the confidence rating may be indicative of the quality or resolution of the tissue image, such as the ability to clearly view the various visual characteristics 212. The confidence rating may indicate a likelihood a predicted polyp characteristics 204 or type classification 202 is associated with the visual characteristic 212. In some examples, the confidence rating may be determined by comparing the similarity, or association between multiple classifications or visual characteristics against each other to validate or invalidate the predictions. For example, detecting the similarity of visual characteristic 212 based on separate inputs or tissue images, or reaching a similar classification prediction based on separate visual characteristics may increase a confidence rating.

In some examples, the machine learned model 112 provides a basis for the various actions of the model. For example, the machine learned model 112 may be trained to provide a basis corresponding to detected or determined visual characteristics 212, classification predictions such as polyp characteristics 204 or type classifications 202, weightings, confidence ratings, or other outputs from the model 112. The basis may be human readable or interpretable information corresponding to the output. For example, the basis may be natural language text providing a description of the detected visual characteristics 212, or associations between the detected visual characteristics 212 and polyp characteristics 204 or type classification 202. The machine learned model 112 may be trained to produce the basis by analyzing the information associated with the labels of the labeled images 117.

In some examples, multiple machine learned models 112 may be included in the system 100, such as on the same or separate servers 110. For example, the multiple machine learned models 112 may generate different classification predictions to provide a comparison between models or predictions.

After training, the machine learned model 112 generates various classification predictions of in vivo polyps captured in tissue images. In some examples, the machine learned model 112 generates a classification prediction, such as a polyp characteristic 204 or type classification 202, of the polyp captured in a tissue image based on the two or more visual characteristics 212. For example, the machine learned model 112 may analyze the visual characteristics 212 to determine an association, weighting, or confidence rating in the analysis to generate the classification prediction. By generating a classification prediction based on multiple visual characteristics 212 of polyps, a more detailed or accurate classification prediction may be generated. In some examples, the machine learned model 112 may generate the basis of the classification prediction to further provide supporting evidence or detail to a medical professional to assist in a diagnosis.

With reference to FIG. 2, a table 200 illustrates example diagnostic information for polyps. The diagnostic information may include type classifications 202 and polyp characteristics 204 that may define the labels of the training images 117. Each of the type classifications 202 or polyp characteristics 204 may be indicated by visual characteristics 212 of the polyps, which may also define the labels for the training or labeled images 117. The classifications of the polyps may be based on the Narrow Band Imaging (NBI) International Colorectal Endoscopic (NICE) Classification system to predict a type based classification of polyps. In some examples, additional characteristics or classifications systems may be used to supplement or replace the NICE classification system of a polyp. By using the NICE system, or other existing classification systems, information included in or generated by the system 100 may fit within current diagnostic processes of medical professionals.

With reference to the visual characteristics 212 of each polyp characteristic 204, table 200 lists example visual characteristics 212 indicative of the type classifications 202 or pathologies 215. The polyp characteristics 204 considered in labelling a training image 117, or for analyzing a tissue image, may be a color 206 of the polyp or tissue area surrounding the polyp. The polyp characteristics 204 may be features or characteristics of vessels 208 of the polyp. The polyp characteristics 204 may be characteristics or features of a surface pattern 210 of the polyps.

The polyp characteristics 204 may be indicative of a type 202 or histopathology 215, as may be indicated by the one or more visual characteristics 212. For example, the visual characteristics 212 associated with one or more polyp characteristics 204 may indicate a type 1 type classification 202 or a hyperplastic histopathology 215. For example, visual characteristics 212 showing a color 206 of a polyp that is the same or lighter than the background tissue area may indicate the type 1 type classification 202 or a hyperplastic histopathology 215. With reference to the visual characteristics 212 of the vessels 208 of a polyp, visual characteristics 212 indicating no vessels, or isolated vessels, may indicate the type 1 type classification 202 or a hyperplastic histopathology 215. With reference to the visual characteristics 212 of the surface pattern 210 of a polyp, surface pattern 210 visual characteristic 212 showing uniform spots (e.g. white or dark spots of a similar size), circular patterns, or an absence of a pattern, which may often be homogeneous, may indicate the type 1 type classification 202 or a hyperplastic histopathology 215. An example depiction of the type 1 type classification or a hyperplastic histopathology may be shown by polyp 222.

The visual characteristics 212 of one or more polyp characteristics 204 may indicate a type 2 type classification 202 or an adenoma histopathology 215. Visual characteristics 212 showing a color 206 of a polyp is darker or browner relative to the background, especially when the color comes from the blood vessels, may indicate the type 2 type classification 202 or an adenoma histopathology 215. Visual characteristics 212 of the vessels 208 of a polyp where the vessels are darker or visible relative to the surrounding tissue area, or that may include surrounding, sometimes white, structures may indicate the type 2 type classification 202 or an adenoma histopathology 215. Surface pattern 210 visual characteristics 212 of a polyp showing branching features or structures surrounded by vessels, often thicker or brown vessels, may indicate the type 2 type classification 202 or an adenoma histopathology 215. In some examples, the type 2 surface pattern 210 visual characteristic 212 may be oval shaped, tubular, or linear branching structures. An example depiction of the type 2 type classification or an adenoma histopathology may be shown by polyp 224.

The visual characteristics 212 of one or more polyp characteristics 204 may indicate a type 3 type classification 202 or a malignant histopathology 215. Visual characteristics 212 showing a color 206 of a polyp is even further darker, such as brown to dark brown, relative to the surrounding tissue area, or that includes lighter (e.g. white) patchy areas may indicate the type 3 type classification 202 or a malignant histopathology 215. Visual characteristics 212 of the vessels 208 of a polyp where the vessels appear distorted (e.g. interrupted, missing, or angled) may indicate the type 3 type classification 202 or a malignant histopathology 215. Surface pattern 210 visual characteristics 212 of a polyp showing a distorted pattern, amorphous or absent pattern, or a depressed area in the polyp may indicate the type 3 type classification 202 or a malignant histopathology 215. An example depiction of the type 3 type classification or a malignant (e.g. cancerous) histopathology may be shown by polyp 226.

In some examples, additional or different polyp characteristics 204, and associated visual characteristics 212, may be considered and analyzed by the system 100. For example, a size, location, previous history of the polyp, a patient's medical history, or other factors may be considered. Similarly, additional type classifications 202, or subsets of the type classifications 202 beyond those depicted in FIG. 2 may be included.

With reference to the training database 115, the various visual characteristics 212, polyp characteristics 204, type classifications 202 and other diagnostic information referenced in FIG. 2 may be stored in the training database 115. The diagnostic information may be stored in the database 115 independent of other information or as labels of the training images 117. For example, the labels of the training images 117 may include information stating a surface pattern polyp characteristic 210 based on a color or size visual characteristic 212, which then may be associated with a type classification 202. In one example, a type 1 classification 202 based on color visual characteristics 212 indicating uniform spots forming a surface pattern 210. In some examples, the diagnostic information represented in table 200 may itself define a training image 117 or training information for use by the system 100.

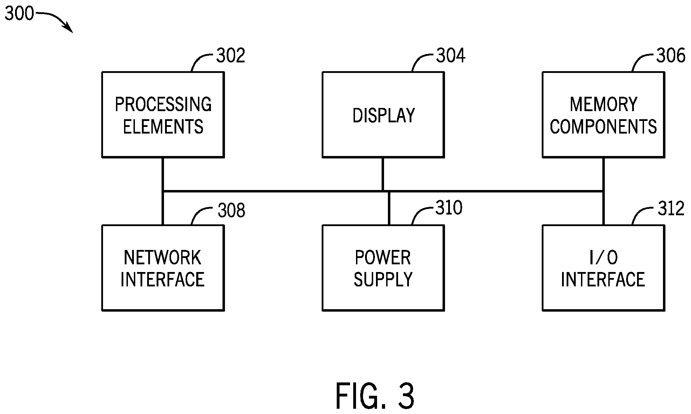

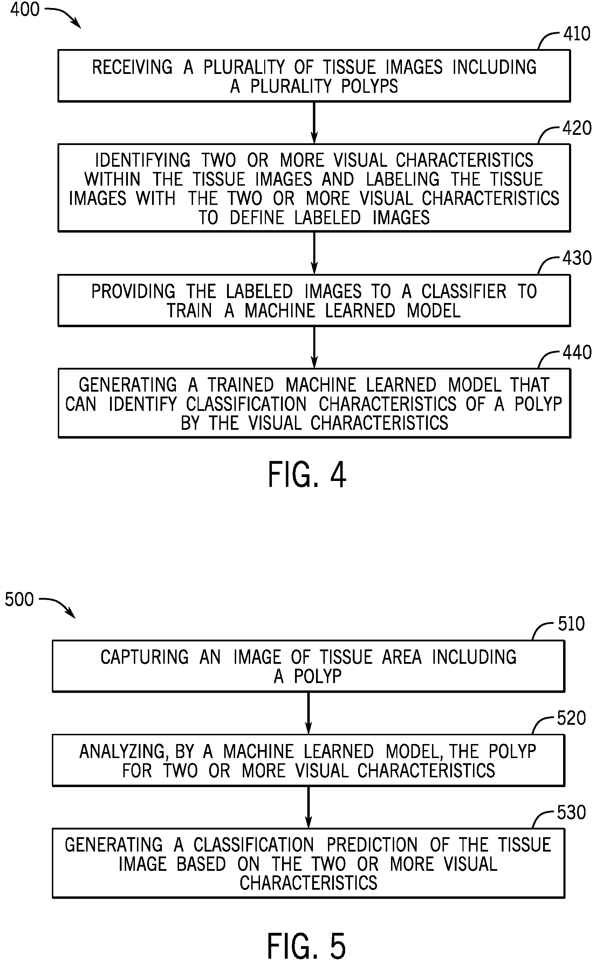

FIG. 3 is a schematic diagram of an example computer system 300 for implementing various systems, such as system 100, and methods, such as methods 400 or 500, in the examples described herein. One or more of the computer systems 300, or portions of the computer system 300, may be included in one or more of the devices or systems disclosed herein. In one example, some or all of the computer system 300 may be included in the server 110. The computer system 300 may be included in, define a portion of, or otherwise in communication with one of more of the training database 115, network 107, or probe 102. The computer system 300 may be used to implement or execute one or more of the components or operations disclosed herein, such as methods 400 or 500 described in FIG. 4 or FIG. 5.

With reference to FIG. 3, the computer system 300 may include one or more of a processing element 302, a display 304, a memory components 306, a network interface 308, a power supply 310, and an input/output (I/O) interface 312. Each of the various components may be in direct or indirect communication with one another through one or more buses, communication networks, such as wired or wireless networks or connections.

The one or more processing elements 302 may be substantially any electronic device capable of processing, receiving, and/or transmitting instructions. For example, the processing elements 302 may be a microprocessor, microcomputer, graphics processing unit, or the like. It also should be noted that the processing elements 302 may include one or more processing elements or modules that may or may not be in communication with one another. For example, a first processing element may control a first set of components of the server 110, probe 102, or training database 115, and a second processing element 302 may control a second set of components of the server 110, probe 102, or training database 115. The first and second processing elements may or may not be in communication with each other. Relatedly, the processing elements 302 may be configured to execute one or more instructions in parallel locally, and/or across the network 107, such as through cloud computing resources. In one example, the processing element 302 may form a portion of the machine learned model 112 (e.g. a neural network) to generate classification predictions, where the machine learned model 112 is in operative communication with the probe 102 to receive the image and trained on the labeled images 117 of a plurality of polyps identified by one or more of the visual characteristics 212.

The display 304 may provide an input/output mechanism for the computing devices, such as to display visual information (e.g., images, graphical user interfaces, videos, notifications, and the like) to the user, and in certain instances may also act to receive user input (e.g., via a touch screen or the like). The display 304 may be a liquid crystal display screen, plasma screen, light emitting diode screen, an organic liquid emitting diode screen, or the like. The type and number of displays may vary with the type of devices (e.g., smartphone versus a desktop computer). In some examples, the classification predictions generated by the machine learned model 112 or the system 100 may be depicted on the display 304.

The memory components 306 store electronic data that may be utilized by the computing devices, such as audio files, video files, document files, programming instructions, and the like. The memory components 306 may be, for example, non-volatile storage, a magnetic storage medium, optical storage medium, magneto-optical storage medium, read only memory, random access memory, erasable programmable memory, flash memory, or a combination of one or more types of memory components. In some examples, the servers 110 or training database 115 may have a larger memory capacity than the computing devices probe 102. The memory components 306 may be optionally linked via a cloud network or the like.

The network interface 308 may receive and transmit data to and from the network 107 to the various devices. The network interface 308 may transmit and send data to or through the network 107 directly or indirectly. For example, the networking interface 308 may transmit data to and from other computing devices through the network 107, which may be a cellular, satellite, or other wireless network (e.g. Wi-Fi, WiMAX, or Bluetooth) or a wired network (e.g. Ethernet), or a combination thereof. In some embodiments, the network interface 308 may also include various modules, such as an API that interfaces and translates requests across the network 107 to the specific local computing elements for the various devices.

The various devices and/or servers may also include a power supply 310. The power supply 310 provides power to various components of the devices. The power supply 310 may include one or more rechargeable, disposable, or hardwire sources (e.g., batteries, power cord, AC/DC inverter, DC/DC converter, or the like). Additionally, the power supply 310 may include one or more types of connectors or components that provide different types of power to the devices and/or servers. In some embodiments, the power supply 310 may include a connector (e.g. a universal serial bus) that provides power to the device or batteries within the device and also transmits data to and from the device to other devices.

The input/output (I/O) interface 312 allows the various devices and/or servers to receive input from a user and provide output to the user. For example, the input/output interface 312 may include a capacitive touch screen, keyboard, mouse, stylus, or the like. The type of devices that interact via the input/output interface 312 may be varied as desired. It should be noted that the various computing devices may be in communication with a compute back end, such as a server or a cloud provider (e.g. Google Cloud Platform, Amazon Web Services, Microsoft Azure, or the like).

To facilitate the reader's understanding of the various functionalities of the embodiments discussed herein, reference is now made to the flow diagram in FIG. 4, which illustrates an example method 400 for training a machine learned model 112 to classify features of a polyp. While specific steps (and orders of steps) of the methods presented herein have been illustrated and will be discussed, other methods (including more, fewer, or different steps than those illustrated) consistent with the teachings presented herein are also envisioned and encompassed with the present disclosure.

At operation, the method 400 may include operation 410 including receiving a plurality of tissue images including a plurality of polyps. The tissue images may be videos or photographs of an in vivo tissue area, such as a colon. The tissue images may each include a single polyp or two or more polyps. The tissue images may depict or have been captured under specific wavelengths of light, or the tissue images may have been filtered during capture or after capture to remove undesired wavelengths of light. The tissue images may be stored in the training database 115. In some examples, the tissue images are captured or stored on a secondary device, which may be separate from the system 100, and transferred to the training database 115, such as by the network 107. The tissue images may be captured under a variety of electromagnetic spectrum, such as visible light, infrared light, or other spectrums of light. For example, the tissue images may be captured by an image capturing device 104 on a variety of types of endoscopic probes 102. In some examples, the tissue images are captured by a device, such as the probe 102, and then initially communicated to an intervening secondary computer or device, then sent to the system 100 by the network 107. For example, the images may be formatted or prepared for use by the system 100 by the secondary device.

The example method 400 may include step 420 including identifying two or more visual characteristics 212 within the tissue images and labeling the tissue images with the two or more visual characteristics to define training or labeled images 117. The labeled images 117 may be stored in and accessible from the training database 115. Each labeled image 117 may include labels readable by devices of the system 100, such as the machine learned model 112, to identify features indicative of the visual characteristics 212. The labels may identify each of a plurality of visual characteristics 212 in a training image 117. In some examples, the labels may identify a subset, or sometimes a single visual characteristic 212, of the plurality of visual characteristics 212 in a training image 117. The labeled images 117 may include labels associating the visual characteristics 212 with classification characteristics such as polyp characteristics 204 or type classifications 202 of a polyp. For example, a visual characteristic 212 related to a color of a tissue may be related to the color polyp characteristic 206 or the vessel structure 208. The labels may similarly associate polyp characteristics 204 with type classifications 202. For example, the labels may indicate brown colors and branching structures indicates a type 2 type classification 202. The association may be defined by including separate labels for each of the visual characteristics 212, the polyp characteristics 204, or type classifications 202 in a single labeled image 117. The association may be defined by or in a single label jointly identifying at least the visual characteristic 212 and one or both of the polyp characteristics 204 or type classifications 202.

The labels of the labeled images 117 may be or include metadata or natural language information of the visual characteristics 212 in the labeled images 117. The labels may be generated by a medical professional or automatically by a separate algorithm. For example, an algorithm for the detection of color may be used to define a subset of labels in the labeled images.

The example method 400 may include operation 430 including providing the labeled images 117 to a classifier to train a machine learned model 112. The classifier may be included or executable by the server 110, or otherwise in communication with the machine learned model 112, such as by the network 107. The machine learned model 112 may similarly access the training images 117 at the training database 115 through the network 107. The machine learned model 112 may be a neural network. For example, the machine learned model 112 may be a multi-label classifier trained to analyze multiple visual characteristics 212 to generate a classification prediction.

During training, the machine learned model 112 may analyze the labeled images 117 and labels included with the images 117 to associate detected visual characteristics 212 with polyp characteristics 204. Similarly, the machine learned model 112 may be trained to associate the visual characteristics 212 or polyp characteristics 204 with type classifications 202 or pathologies 215 of a polyp. In some examples, the machine learned model 112 may be trained to analyze patterns or associations between two or more visual characteristics 212 to improve identification of polyp characteristics 204 or type classifications 202 of polyps. For example, the machine learned model 112 may link or associate commonly appearing visual characteristics 212 as additional characteristics indicative of a polyp characteristic 204 or a type classification 202 of a polyp. The machine learned model 112 may analyze detected or determined polyp characteristics 204 to determine type classifications 202.

The machine learned model 112 may be trained to weight a certain set of visual characteristics 212 as more indicative of a polyp characteristic 204 or a type classification 202 over additional visual characteristics 212. In some examples, the machine learned model 112 may be trained to weight polyp characteristics 204, or associated polyp characteristics 204, as indicative of a type classification 202. For example, for some polyps, the machine learned model 112 may learn from the training data that visual characteristics 212 identifying a color 206 and vessel structures 208 may be more indicative of one of the type classifications 202 over a surface pattern 210 indicative of a separate type classification 202. The weighting may be a mathematical expression or numerical factor assigned to the visual characteristics 212 or polyp characteristics 204.

In some examples, the machine learned model 112 may be trained to provide a confidence rating in a classification prediction. For example, the machine learned model 112 may provide a confidence rating indicative of a similarity of a detected visual characteristic 212 to a similar or same visual characteristic 212 in the training images 117. In some examples, the confidence rating may indicate a likelihood that a visual characteristic 212, or a collection of visual characteristics 212, is associated with polyp characteristic 204 or type classification 202. The confidence rating may be expressed as a percentage, ratio, or numerical value that may be interpreted as representative of the likelihood of a correct classification or detection of a visual characteristic 212.

In some examples, the machine learned model 112 may be trained to associate treatment options or recommendations with the classification characteristics. The treatment recommendations may be included as labels in the labeled images 117. The machine learned model 112 may be trained to identify the treatment options by similar collections or groupings of classification characteristics such as a collection of visual characteristics 212, polyp characteristics 204, or type classifications 202.

The example method 400 may include step 440 including generating a trained machine learned model 112 that can identify classification characteristics by the visual characteristics 212. The classification characteristics may include or correspond to one or more of the polyp characteristics 204 or type classifications 202 of a polyp. For example, the classification characteristics may correspond to the NICE classification system or another known classification system to fit within a medical professional's existing diagnostic process. The classification characteristics may include a basis or descriptions of one or more of the visual characteristics 212, polyp characteristics 204, or type classifications 202 of a polyp. For example, the trained machine learned model 112 may generate a basis including natural language text or other human readable outputs to describe the detected features of a polyp. The trained machine learned model 112 may be trained to generate the basis by analyzing the format or content of the labels indicating the visual characteristics 212, polyp characteristics 204, or type classifications 202 of a polyp. The basis may assist medical professionals in interpreting the classification characteristics and increase the reliability and usefulness of the classification characteristics.

In some examples, generated classification characteristics or classification predictions of the trained machine learned model 112 may be validated and added to the training images 117, or otherwise communicated to the trained machine learned model 112 to further improve classification predictions.

To facilitate the reader's understanding of the various functionalities of the embodiments discussed herein, reference is now made to the flow diagram in FIG. 5, which illustrates an example method 500 for analyzing a tissue image including a polyp, by a machine learned model 112, to classify features of a polyp or generate a classification prediction of a polyp. While specific steps (and orders of steps) of the methods presented herein have been illustrated and will be discussed, other methods (including more, fewer, or different steps than those illustrated) consistent with the teachings presented herein are also envisioned and encompassed with the present disclosure.

The example method 500 may include operation 510 including capturing an image of a tissue area including a polyp. The image may be captured by a device, such a probe 102. For example, the probe 102 may include an image capturing device 104 to capture tissue in vivo tissue images. The in vivo location may be an interior or wall of a colon or intestine. Accordingly, the probe 102 may be inserted into the body to reach the in vivo location. The tissue image may be a single image or a plurality of images. In some examples, two or more tissue images may be captured by two or more image capturing devices 104. The tissue image may be videos or photographs. The tissue images may be a capture of the visible light spectrum, or a variety of other spectrums useful in detecting visual characteristics 212 of a polyp. For example, a light source may generate a variety of wavelengths of lights and illuminate a tissue area for capture by the image capturing device 104.

The tissue images may be captured continuously during a colonoscopy or the captured at defined locations. In some examples, the system 100, such as the machine learned model 112, may detect the presence of a polyp in the tissue image. In such an example, the machine learned model 112 may communicate with the image capturing device 104 to capture tissue images when the polyps are in view of the image capturing device 104. In some examples, a medical professional may analyze the images to determine when a polyp is included to define a tissue image. The tissue images may be analyzed in real time, such as during a colonoscopy, or analyzed after the completion of the procedure. In some examples, the tissue images may be captured by a secondary device or a device separate from the system 100. The tissue images may then be communicated to the system 100, such as by the network 107, for analysis. After capturing the tissue images including polyps, the tissue images may be communicated, such as by the network 107, to the machine learned model 112 for analysis.

Instead of operation 510 including capturing an image of a tissue area including a polyp, operation 510 may alternatively include receiving an image of a tissue area including a polyp. For example, where the tissue images are captured by a secondary device or a device separate from the system 100, the captured images may be received at the system 100, such as over the network 107, for analysis. That is, in vivo tissue images may be captured at a first point in time by a separate probe or image capturing device (e.g., during a colonoscopy procedure). Then, at a second point in time, the captured images may be communicated to the system 100 for analysis.

The example method 500 may include operation 520 including analyzing, by a machine learned model 112, the polyp for two or more visual characteristics 212. The machine learned model 112 may analyze the polyp or the surrounding tissue area in the tissue image to detect the two or more visual characteristics 212 of the polyp. As discussed herein, the machine learned model 112 may be a multi-label classifier and may interpret multiple inputs in the form of a plurality of visual characteristics 212. The machine learned model 112 may compare the tissue image to the training images 117, such as the labels of the training images 117, to detect the visual characteristics 212. For example, the machine learned model 112 may look for matching or corresponding visual characteristics 212. In some examples, the machine learned model 112 may compare the tissue image to patterns determined during training to be indicative of visual characteristics 212 rather than, or in addition to, the labeled visual characteristics 212. In some examples, the machine learned model 112 may predict the location of various visual characteristics 212 to determine which regions of the tissue image to analyze. For example, the presence of a color change may be indicative of a presence of vessels in a polyp.

During analysis the machine learned model 112 may begin with seeking weighted characteristics or visual characteristics 212 more likely to be indicative of a classification characteristic, such as polyp characteristics 204 or type classifications 202, or indicative of what other visual characteristics 212 may be present. For example, a vessel color visual characteristic 212 may be a characteristic indicative of each of visual characteristics 212, polyp characteristics 204, or a type classification 202 and the machine learned model 112 may analyze the tissue image accordingly. In some examples, a plurality of visual characteristics 212 may be detected. In such an example, all or some of the visual characteristics 212 may be noted or stored for additional analysis. For example, the visual characteristics 212 may be sorted by those having a higher weight, or by those having a confidence rating deemed sufficient to include in further analysis.

The analysis by the machine learned model 112 may occur continuously or on demand. For example, continuous analysis of the tissue images may occur during a procedure or as the captured images are communicated to the machine learned model 112. On demand analysis may be triggered responsive to an input to the system 100 by a medical professional, such as after the completion of a procedure, during a procedure, or after a certain number of tissue images are captured.

The example method 500 may include operation 530 including generating a classification prediction of the tissue image based on the two or more visual characteristics 212. The classification prediction may correspond to a known classification system, such as the NICE system, to correspond to a medical professional's existing diagnostic process. The classification prediction may be a polyp characteristic 204, type classification 202, or histopathology 215 based on the two or more visual characteristics 212. In some examples, a polyp characteristic 204 determined by the visual characteristics 212 may determine the type classification 202. The classification prediction may include two or more predictions.

The classification prediction may include a type classification 202 for each polyp characteristic 204 or visual characteristic 212, or a single type classification 202 may be based on a plurality of polyp characteristics 204 or visual characteristics. For example, each visual characteristic 212 or polyp characteristic 204 may be indicative of one or more type classifications 202. By generating a classification prediction including a type classification 202 for each polyp characteristic 204 or visual characteristic 212, the classification prediction provides a more comprehensive and flexible insight to a polyp's condition. For example, a first polyp characteristic 204 or visual characteristic 212 may indicate a certain type classification 202, while a second and third polyp characteristic 204 or visual characteristic 212 may indicate a different type classification 202. By providing each classification prediction, a reviewing medical professional may be able to more easily determine or weigh the competing type classifications 202. The comprehensive and flexible insight may better assist a medical professional in diagnosing or treating a polyp.

The classification prediction may include a basis, or description of the classification prediction including a description of one or more of the polyp characteristics 204, type classifications 202, histopathology 215, the two or more visual characteristics 212 relied on, or other factors used to generate the classification prediction of a polyp in the tissue image. The basis may be generated by analyzing labels of training images 117 including similar features to those detected or determined by the polyp. In some examples, the basis is in the form of a natural language text or human interpretable description. By providing an understandable description of the classification prediction, a medical professional will be provided additional insight into the polyp and may more reliably corroborate or validate the classification prediction.

In some examples, the machine learned model 112 may generate a classification prediction based of a confidence, such as confidence rating, in the detected visual characteristics 212 or in the determination of the polyp characteristics 204, type classifications 202, or histopathology 215 based on the two or more visual characteristics 212. For example, a first set of visual characteristics 212 (e.g. one or more visual characteristics 212) may indicate a first type classification 202, while a second set of visual characteristics 212 (e.g. one or more different visual characteristics 212) may indicate a different type classification 202. In such an example, the machine learned model 112 may determine the type classification 202 based on a confidence rating of the visual characteristics 212 or each set of visual characteristics 212. For example, a type classification 202 based on visual characteristics 212 with a high confidence rating in the detection or analysis of the visual characteristic 212 may be deemed more accurate than a type classification 202 based on visual characteristics 212 with a lower confidence rating. The confidence rating of the various features considered in the classification prediction may be included in the basis.

In some examples, the machine learned model 112 may weight visual characteristics 212 or polyp characteristics 204 that are commonly more indicative of a type classification 202 or polyp characteristics 204. The weight may be included in or influence the classification prediction. For example, either of a first set of visual characteristics 212 or corresponding polyp characteristics 204 may be treated as determinative, or more determinative, over a second set in generating a classification prediction. Accordingly, a corresponding type classification 202 or polyp characteristics 204 to the first set may be weighted over the second set. In some examples, groupings of visual characteristics 212 or polyp characteristics may more indicative of a type classification 202 and assigned a corresponding weight as a group. The weighting may be determined during training or as an input to the system. In some examples, the machine learned model 112 may provide only a recommendation corresponding to the weighting for review by a medical professional, but otherwise produce an unweighted classification prediction.

In some examples, the classification prediction may include a recommended diagnosis or treatment. The diagnosis or treatment may be based on one or more of the detected visual characteristics 212 or on the determination of the polyp characteristics 204, type classifications 202, or histopathology 215. In some examples, the diagnosis or treatment may be generated by comparing diagnosis and treatments of corresponding polyps in the labeled images 117 to the similarity of the detected visual characteristics 212, polyp characteristics 204, type classifications 202, or histopathology 215 of a polyp in the tissue images.

In some examples, the classification prediction may be validated and added to the training images 117 or otherwise used to assist in improving the machine learned model 112. For example, the classification prediction may be validated by a medical professional, further testing, or other reliable methods.

In some examples, the classification prediction is depicted on a display, such as on a display 304. The display of the classification prediction may be during the capture or analysis of the tissue image, or otherwise in real time during a procedure, or at a later time after a procedure or analysis. The display may include each of the features described herein or subsets of the information. For example, various medical professionals may be concerned about a specific set or grouping of the visual characteristics 212, polyp characteristics 204, type classifications 202, or histopathology 215. Accordingly, the corresponding information may be displayed.

By providing a system 100 that may be trained to detect and generate classification predictions based on multiple visual characteristics 212 of a polyp, medical professionals may be provided increased amounts of information to improve diagnosis accuracy and/or decrease the time to arrive at a diagnosis to improve patient outcomes.

Other examples and implementations are within the scope and spirit of the disclosure and appended claims. For example, features implementing functions may also be physically located at various positions, including being distributed such that portions of functions are implemented at different physical locations. Also, as used herein, including in the claims, "or" as used in a list of items prefaced by "at least one of indicates a disjunctive list such that, for example, a list of "at least one of A, B, or C" means A or B or C or AB or AC or BC or ABC (i.e., A and Band C). Further, the term "exemplary" does not mean that the described example is preferred or better than other examples.

The foregoing description, for purposes of explanation, uses specific nomenclature to provide a thorough understanding of the described embodiments. However, it will be apparent to one skilled in the art that the specific details are not required in order to practice the described embodiments. Thus, the foregoing descriptions of the specific embodiments described herein are presented for purposes of illustration and description. They are not targeted to be exhaustive or to limit the embodiments to the precise forms disclosed. It will be apparent to one of ordinary skill in the art that many modifications and variations are possible in view of the above teachings.

## Claims

1. A method of classifying a polyp comprising:
capturing a tissue image of an in vivo tissue area including a polyp during a colonoscopy procedure;
analyzing, by a trained machine learned model, the tissue image, wherein the trained machine learned model is trained to identify classification characteristics of a polyp based on two or more visual characteristics; and
generating a classification prediction of the tissue image based on the two or more visual characteristics including a basis of the classification prediction.

2. The method of claim 1, wherein:
the classification characteristics include one or more polyp characteristics; and
the classification prediction includes a type classification for each of the one or more polyp characteristics.

3. The method of claim 2, wherein the one or more polyp characteristics comprise one or more of:
a color of the polyp or the tissue area in the tissue image, vessel features of the polyp, or a surface pattern of the polyp.

4. The method of any of claims 1 to 3, further comprising:
validating the classification prediction to further train the trained machine learned model.

5. The method of any of claims 1 to 4, wherein:
the basis comprises one or more of a confidence rating, a weighting of the classification characteristics, or a human readable description of the classification prediction.

6. The method of any of claims 1 to 5, wherein the trained machine learning model is a neural network.

7. The method of any of claims 1 to 6, wherein the classification prediction includes a recommended treatment including one or more of a biopsy of the polyp or a removal of the polyp.

8. The method of any of claims 1 to 7, further comprising a training method for the machine learned model to classify features of a polyp, the training method comprising:
receiving a plurality of training tissue images including a plurality of polyps;
identifying one or more of a plurality of visual characteristics within the training tissue images and labeling the training tissue images with the one or more of the plurality of visual characteristics to define labeled images; and
providing the labeled images to a classifier to train the machine learned model to generate the trained machine learned model that can identify classification characteristics of the polyp by the two or more visual characteristics.

9. The method of claim 8, wherein a set of the labeled images includes labels identifying one or more polyp characteristics or one or more type classifications associated with the one or more of the plurality of visual characteristics.

10. The method of any of claims 8 or 9, wherein training the machine learned model further comprises training the machine learned model to recognize a presence of the polyp at the tissue area included in the training image.

11. The method of any of claims 1 to 10, wherein:
the two or more visual characteristics are identified as multi-label classifications of the tissue image.

12. The method of any of claims 1 to 11, further comprising:
training the trained machine learned model to weight or prioritize one or more of the classification prediction of the polyp, the classification characteristics, or the two or more visual characteristics.

13. The method of any of claims 1 to 12, wherein the method is performed by a system comprising:
a probe comprising an imaging device, wherein the imaging device captures the tissue image of the tissue area including the polyp; and
a processing element forming a portion of a neural network to generate classification predictions, wherein the neural network is in operative communication with the probe to receive the tissue image.

14. The method of any of claims 1 to 13, wherein the classification prediction includes two or more type classifications, each of the two or more type classifications based on separate sets of the two or more visual characteristics.

15. A system for classifying a polyp, the system comprising:
a probe including an imaging device, wherein the imaging device is configured to capture an image of a tissue area including the polyp; and
a processing element forming a portion of a trained machine learned model to generate classification predictions, wherein the trained machine learned model is in operative communication with the probe to receive the image and wherein the trained machine learned model is trained on labeled images of a plurality of polyps identified by one or more visual characteristics, wherein the classification predictions are generated by:
identifying each of a plurality of visual characteristics of the polyp in the image;
producing a classification prediction corresponding to the plurality of visual characteristics of the polyp, wherein the classification prediction includes a basis of the classification prediction; and
displaying the classification prediction.
